# EUROPEAN PATENT SPECIFICATION

(11) **EP 3 469 086 B1**
(45) Date of publication and mention of the grant of the patent: **01.06.2022**
(21) Application number: 17748569.5
(22) Date of filing: 13.06.2017
(51) Int. Cl.: C12P 5/02, C02F 11/04, C02F 11/18, C12M 1/107

(54) **PROCESS FOR TREATING AND GENERATING ENERGY FROM BIOMASSES**
VERFAHREN ZUR BEHANDLUNG UND ENERGIEERZEUGUNG AUS BIOMASSEN
PROCÉDÉ DE TRAITEMENT ET DE PRODUCTION D'ÉNERGIE À PARTIR DE BIOMASSES

(30) Priority: 14.06.2016 EP 16174427; 07.09.2016 IT 201600090522
(43) Date of publication of application: 17.04.2019
(73) Proprietor: Geo Environmental Energy Technologies S.r.l., 70126 Bari (BA) (IT)
(72) Inventor: PASTORE, Nicola, 70010 Casamassima (BA) (IT); GIASI, Concetta Immacolata, 70044 Polignano a Mare (BA) (IT)
(74) Representative: Perani & Partners S.p.A.
(86) International application number: PCT/IB2017/053502
(87) International publication number: WO 2017/216720

(56) References cited:
- WO-A1-88/04282
- W W CLARKSON ET AL: "Bench-scale anaerobic bioconversion of newsprint and office paper", WATER SCIENCE AND TECHNOLOGY, vol. 41, no. 3, 1 January 2000 (2000-01-01), pages 93-100, XP055366973, ISSN: 0273-1223
- PARK C ET AL: "Upgrading of anaerobic digestion by incorporating two different hydrolysis processes", JOURNAL OF BIOSCIENCE AND BIOENGINEERING, ELSEVIER, AMSTERDAM, NL, vol. 100, no. 2, 1 August 2005 (2005-08-01), pages 164-167, XP027707227, ISSN: 1389-1723 [retrieved on 2005-08-01]
- Katerina Stamatelatou ET AL: "The Effect of Physical, Chemical, and Biological Pretreatments of Biomass on its Anaerobic Digestibility and Biogas Production" In: "Biogas Production, Pretreatment methods in anaerobic digestion", 1 January 2012 (2012-01-01), John Wiley & Sons, Inc, Hoboken, NJ, USA, XP055367916, ISBN: 978-1-118-06285-2 pages 55-90, DOI: 10.1002/9781118404089.ch3, page 68, paragraph 2 - page 69, paragraph 2 page 81, paragraph 2
- NEUMANN P ET AL: "Developments in pre-treatment methods to improve anaerobic digestion of sewage sludge", REVIEWS IN ENVIRONMENTAL SCIENCE AND BIO-TECHNOLOGY, KLUWER, DORDRECHT, NL, vol. 15, no. 2, 3 May 2016 (2016-05-03), pages 173-211, XP035930349, ISSN: 1569-1705, DOI: 10.1007/S11157-016-9396-8 [retrieved on 2016-05-03]

## Description

### Field of the invention

The present invention relates to a process for treating and generating energy from biomasses, through anaerobic digestion. Furthermore the present application discloses a plant in which said method for treating biomasses can be implemented.

### Priorart

At the state of the art, anaerobic stabilisation is used for energy valorisation of biomasses.

Anaerobic digestion represents a valid process for energy valorisation and for the consequent biomasses disposal, but it has the limitation of being expensive and bulky.

According to state of the art a method to increase the efficiency of anaerobic digestion is to thicken the moist biomass increasing the total solids content. This method could be inconvenient both for the costs related to the thickening and for the progressive decrease of the anaerobic digestion efficiency as the volatile solids content increases.

In the scientific and technical literature several methods to enhance the digestibility of biomass exist. The most interesting approaches are based on thermal treatment, enzymatic treatment, mechanical disintegration, ultrasonication and thermochemical hydrolysis.

The former appears to be very promising since permits to solubilize up to 70% of the total solids forming the biologic sludges.

The thermochemical hydrolysis, according to the state of the art, is performed in acid (pH 1-3) or alkaline catalytic condition (pH 11-13) at temperatures comprised between 60°C and 120°C and with a treatment time normally ranging between 1 hour and 6 hours.

Experimental evidences show that, as the total solid content increases along with the volatile solids present in the biomass, the efficiency of the thermochemical hydrolysis treatment decreases significantly and the anaerobic digestibility of the biomass diminishes consequently, reducing the energy efficiency of the process.

WO8804282 A1 discloses a method for the restructuring of wastewater treatment sludges and conditioned solid wastes to remove non-biodegradable solids and to convert biodegradable matter into methane fuel gas. This process for treating and generating energy from biomass includes the steps of: i. providing a biomass; ii. optionally diluting the biomass with a thinner; iii-1. optionally subjecting the sludge to particle size reduction; iii-2. subjecting the sludge to enzymatic hydrolysis; iii-3. Alkalizing the biomass of step iii-2 with a basic solution; iv. hydrolyzing the mixture under application of heat, thereby obtaining a hydrolyzed biomass; v. neutralizing the hydrolyzed biomass through diffusion of CO₂ from a biogas produced by anaerobic digestion, thereby obtaining a hydrolyzed and neutralized sludge; vi. Performing anaerobic digestion, through a first anaerobic digestor of said hydrolyzed and neutralized sludge, thereby obtaining a biogas, a first digestate and an anaerobic sludge.

Clarkson et al. (W.W. Clarkson, X. Xiao; Bench-scale anaerobic bioconversion of newsprint and office paper. Water Sci Technol 1 February 2000) disclose a process for the bioconversion of newsprint and waste office paper, performed in bench-scale reactors, with three inocula sources: landfill, rumen and anaerobic digester.

Park C. et al (Chulhwan Park, Chunyeon Lee, Sangyong Kim, Yu Chen, Howard A. Chase, Upgrading of anaerobic digestion by incorporating two different hydrolysis processes, Journal of Bioscience and Bioengineering, Volume 100, Issue 2, 2005, pages 164-167) describe a study aimed at investigating how to increase the efficiency of anaerobic digestion of waste activated sludge (WAS). Either thermochemical or biological hydrolysis was used as a pretreatment and the effects of both were investigated and compared. Two different three-stage digestion systems showed improved performance, although thermochemical hydrolysis showed better results than biological hydrolysis in a bench-scale operation. After anaerobic digestion with thermochemical pretreatment, the total chemical oxygen demand (tCOD) reduction, volatile solid (VS) reduction, methane yield and methane biogas content were 88.9%, 77.5%, 0.52 m3/kg VS and 79.5%, respectively.

Stamatelatou K. et al (Stamatelatou, Katerina & Antonopoulou, G. & Ntaikou, Ioanna & Lyberatos, Gerasimos. (2013). ChemInform Abstract: The Effect of Physical, Chemical, and Biological Pretreatments of Biomass on Its Anaerobic Digestibility and Biogas Production. Chemlnform. 44) disclose several pretreatment methods developed to render organic feedstocks more amenable to anaerobic digestion. Two kinds of feedstocks require the application of pretreatment to enhance the anaerobic biodegradability: those containing lignocellulosic polymers (plant biomass, etc.) and those containing microbial-type polymers (activated sewage sludge, etc.). The efficiency of the method is evaluated with respect to the increase in the biogas produced in the subsequent step of the anaerobic digestion, and the other objectives of the overall treatment configuration.

Neumann P. et al (Neumann, P., Pesante, S., Venegas, M. et al. Developments in pre-treatment methods to improve anaerobic digestion of sewage sludge. Rev Environ Sci Biotechnol 15, 173-211 (2016)) disclose pre-treatments to hydrolyze sludge, and consequently improve biogas production, solids removal and sludge quality after digestion The study is an overview of different technologies, discussing their effects on sludge properties and anaerobic digestion.

### Summary of the invention

The Applicant has now discovered that is possible to significantly increase the anaerobic digestibility of the biomass through optimisation of the solubility of the Chemical Oxygen Demand (COD), with a low energy consumption.

According to the present invention, this purpose is achieved through a method for treating and generating energy from biomasses according to claim 1.

The process according to the present invention allows the production of a biogas (biomethane) with a high purity grade, with a CO2 content lower than 0,2%, and an improvement of the energy efficiency of the biomass treatment process, up to a performance efficiency greater than 100%.

Furthermore, the application discloses a biomass treatment plant, which is not part of the present invention, wherein the method according to the present invention can be implemented.

### Description of the figures

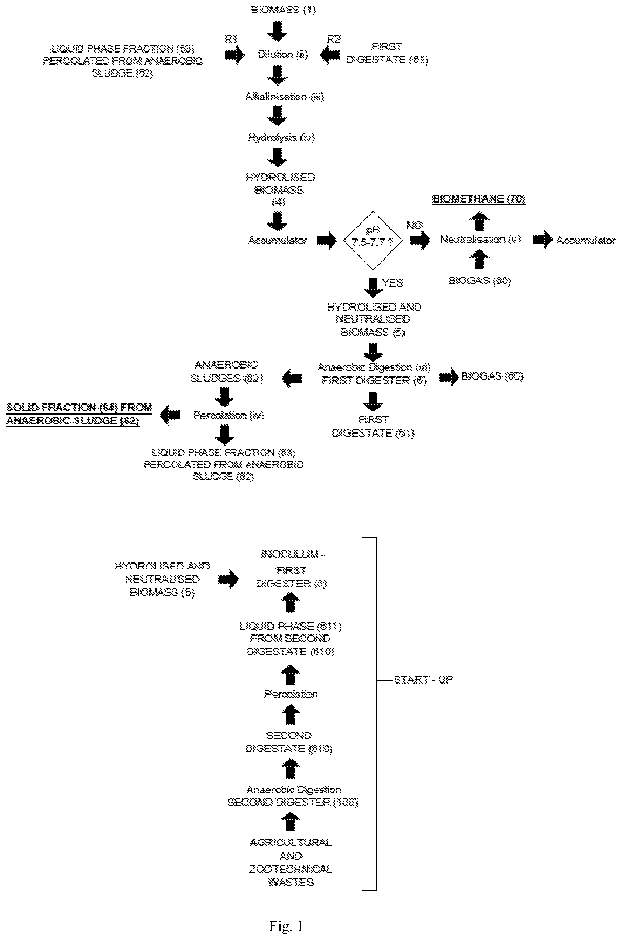

Figure 1: Flow chart representing the main steps and the products obtained through the treatment of biomass 1 according to the present invention. Figure 1 represents two preferred embodiments of the process according to the invention wherein the dilution ii of the biomass 1 is performed recycling the fraction of liquid phase 63 percolated from the anaerobic sludge 62 or recycling the first digestate 61. The recycling ways displayed in figure 1 with symbols R1 or R2 are therefore alternative. Furthermore Figure 1 shows the production cycle of the liquid phase 611 of the second digestate 610, wherein said liquid phase 611 of the second digestate 610 is used as inoculant to trigger the anaerobic digestion vi in the first anaerobic digester 6.

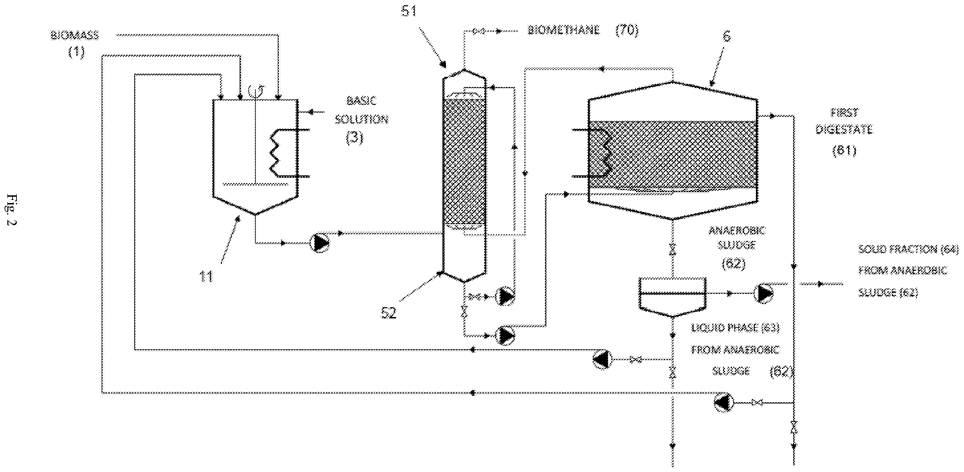

Figure 2: Scheme of a plant for the treatment of biomasses according to the method claimed in the present invention, comprising a hydrolysis reactor 11, a column reactor 51 comprising an accumulator 52 and a first digester 6 in fluid communication. The hydrolysis reactor 11 is equipped with a heating system (such as an electrical resistance). The column reactor 51 is equipped with a recirculation system that re-inject the material to be neutralised at the top of the neutralisation column. The first anaerobic digester 6 is equipped with a heating system (such as electrical resistance). The embodiment displayed in Figure 2 shows even the recirculation system of the first digestate 61 and of the liquid fraction 63 of the anaerobic sludge 62, suitable for their respective re-injection into the hydrolysis reactor 11.

### Detailed description of the invention

With reference to enclosed Figure 1, the main steps of the process according to the present application are schematically represented in a flow chart, where with biomass 1 is indicated a substrate suitable for transforming and generating energy according to the present invention.

Generally, by biomass is meant any material having a biodegradable fraction of biological origin coming from forestation and agriculture, comprising both vegetable and animal substances; any product, waste and leftover from woodworking and paper processing industry; any organic product, waste and leftovers having a biodegradable fraction derived from human and animal biological activity, such as those contained in industrial and urban waste.

Within the aims of the present invention, said biomass 1 is chosen among at least one of the following substrates: biologic sludges, organic wastes, process water, treated or not treated residual sludges from the plants for treating urban, household and industrial wastewater, treated or not treated residual sludges from production processes, wherein the organic wastes are organic wastes resulting from compost plants, comprising wastes derived from agriculture, horticulture and food preparation, sludges resulting from civil and industrial wastewater treatment plants. Within the aims of the present invention by biologic sludge is meant the sludges produced from the urban and/or industrial wastewater characterized by high moisture content.

By process waters is meant wastewater obtained from agroindustrial processes, such as the vegetation waters resulting from the production of olive oil, the milk whey resulting from cheese making processes, or the washing effluents resulting from wine processing. By treated or not treated residual sludges from the plants for treating urban, household and industrial wastewater is meant the sludges produced from the purification processes of urban and household wastewaters; the sludges coming from septic tanks; the sludges coming from the agroindustrial wastewater treatment plants. By treated or not treated residual sludges from production processes is meant the sludges resulting from food farming origin; from paper and wood processing; sludges resulting from textile and leather processing; sludges resulting from oil industry or derived from organic and inorganic chemical processes.

The process object of the present application ensures advantageously to treat both humid biomasses and dry biomasses (with low moisture content), wherein by humid biomass is meant a biomass having a moisture content between 99% and 95% by weight; wherein by dry biomasses (with low moisture content) is meant a shovelable biomass with a moisture content lower than 75%.

In a preferred embodiment, said biomass 1 is formed by treated or not treated residual sludges resulting from the plants for treating urban wastewater.

According to the present invention, the biomass 1 is subjected to a dilution step ii by means of a thinner up to a volatile solids content comprised between 0,5% and 1% of the volume of said biomass 1.

Preferably, the biomass 1 and the thinner are combined, in the dilution step ii, in order to obtain a percentage of the volatile solids between 0,5% and 1% of the volume of said biomass 1.

Advantageously, said dilution step ii allows obtaining, by means of the following steps of alkalising iii and hydrolysing iv, a higher solubilisation of the solids contained in the biomass, solubilisation that increases the digestibility of the material starting the anaerobic digestion step and therefore the energy performance of the process.

The thinner is an aqueous liquid, wherein by aqueous liquid is meant a liquid comprising an amount of water higher than 50% by weight on the total weight of the liquid. Note that, according to an alternative embodiment, water is used as thinner.

In the purpose of the present invention, by COD (Chemical Oxygen Demand) is meant the quantity of oxygen (mg 02/1) needed for the complete oxidation of the organic and inorganic compounds contained in a water sample.

The diluted biomass 1 is subjected, using an alkaline (basic) solution, to an alkalising step iii, up to a pH between 11,0 and 13,0, wherein said diluted biomass 1 and said basic solution 3 are in weight on weight ratio comprised between 1:20 and 1:80.

The basic solution 3, within the purpose of the present invention, is preferably a strong base solution at 20% (weight/volume). Even more preferably, said alkaline solution 3 is a sodium hydroxide solution at 20% (weight/volume).

The biomass 1 thus alkalised, is subjected to a hydrolysis step iv for a time comprised between 30 and 90 minutes, at temperature comprised between 55°C and 80°C.

Advantageously, the conducted hydrolysis step iv allows, with an energy consumption between 0.95 and 2.85 KWh for meter cube of biomass, to generate a hydrolysed biomass 4 characterized by a solubilisation efficiency of the solids contained within the biomass up to 90%.

The solubilisation efficiency of the solids contained within the biomass is measured in terms of ratio between the COD, determined on the clear fraction (supernatant) obtained by filtration and centrifugation of the hydrolysed material, and the total COD of the hydrolysed material.

The hydrolysed biomass 4 has a pH comprised between 10,0 and 13,0 unit of pH. Subsequently, the hydrolysed biomass 4 is subjected to a neutralization step v of the pH, preferably reaching a value of pH between 6,0 and 8,0 unit of pH.

Even more preferably said neutralization step v is conducted until said hydrolysed biomass 4 reaches a value of pH between 7,5 and 7,7.

The neutralization step v is performed by means of CO2 diffusion from a biogas produced from an anaerobic digestion process, towards the hydrolysed biomass 4 resulting from the hydrolysis step iv.

Preferably, the carbon dioxide diffusion in the neutralization step v is performed in a column reactor 51, saturated with the biogas obtained by anaerobic digestion, and filled with inert material, preferably polyethylene pellet, characterized by a porosity preferably greater than 0.5 and a specific surface preferably between 140 and 700 m-1.

Within the column reactors 51, the biogas is kept at a pressure of 0.05 bar.

The column reactor 51 wherein the neutralisation step v occurs is equipped with an accumulation tank 52 (accumulator) which collects the hydrolysed biomass 4 and provides it on top of the neutralization column (column reactor 51).

The hydrolysed biomass 4 feeds the column reactor 51 from the top and it is preferably recirculated with descending flow, by gravity (from top to bottom), until its complete neutralisation.

A hydrolysed and neutralised biomass 5 is obtained from the neutralisation step v, wherein said hydrolysed and neutralised biomass 5 is then sent to a first anaerobic digester 6 in order to undergo the anaerobic digestion step vi.

From the anaerobic digestion step vi of the hydrolysed and neutralised biomass 5, three products are obtained: the biogas 60, the first digestate 61 and the anaerobic sludge 62.

In a preferred embodiment of the process according to the present invention, the neutralisation step v is conducted by means of diffusion of carbon dioxide from the biogas 60, produced by the anaerobic digestion step vi, towards the hydrolysed biomass 4.

When the biogas 60 is used for the neutralisation step v of the neutralised and hydrolysed biomass 5, the neutralisation step has the dual aim of purifying the biogas 60 from carbon dioxide and at the same time of neutralising the hydrolysed and neutralised biomass 5, so that it can be used from the following anaerobic digestion step vi.

The biogas 60, when employed during the neutralisation step v of the hydrolysed and neutralised biomass 5 and purified from the carbon dioxide contained in it, gives rise to a biomethane 70, preferably characterised by a percentage of carbon dioxide lower than 0.2%.

Due to the high digestibility of the hydrolysed and neutralised biomass 5 entering the first anaerobic digester 6, the production yield of the biogas 60 from the anaerobic digestion step vi is particularly advantageous, characterised by a residence time averagely equal to 2 days and a high energy efficiency.

The material at the outlet of the anaerobic digester 6 is constituted by:
i. A digestate 61 that presents a reduction of the volatile solids equal to 90% compared to the inlet biomass;
ii. An anaerobic sludge 62 which, after simple percolation, presents a moisture content also lower than 15%.

The digestate 61 at the outlet of the anaerobic digester can be sent to purification by means of biological oxidation or advantageously utilised for fertigation.

The anaerobic sludge 62 can be sent to disposal or advantageously utilised for energy or agronomic valorisation.

In a first preferred embodiment, said first digestate 61 is the diluent used within the process according to the present invention.

In a second preferred embodiment, the process according to the present patent application is characterized by a further percolation step of the anaerobic sludge 62, wherein a liquid phase 63 percolated from the anaerobic sludge 62 and a solid fraction 64 from the anaerobic sludge 62 are obtained. According to said second preferred embodiment, said liquid phase 63 percolated from the anaerobic sludge 62 is used as a diluent.

In a further embodiment, the anaerobic sludge 62 is used as inlet biomass in the process object of the present patent application. Even more preferably, the trigger of said anaerobic digestion step vi is performed by inoculation of a liquid phase 611 of a second digestate 610 within the first anaerobic digester 6.

Said liquid phase 611 of the second digestate 610 is preferably obtained through an anaerobic digestion of a second anaerobic digester 100 fed with agricultural and zootechnical wastes.

The second digestate 610 thus obtained is preferably percolated in order to obtain a liquid phase 611 of said second digestate 610.

In the preferred embodiment of the process object of the present invention, the liquid phase 611 of the second digestate 610 is inoculated in the first anaerobic digester 6, so that said liquid phase 611 of the second digestate 610 is in a volumetric ratio between 2:1 and 1:2 with the hydrolysed and neutralised biomass 5.

The present application discloses also a plant for treating and generating energy from biomasses according to the method of the present invention; the plant, as such, is not part of the present invention.

In particular, said plant is characterized by comprising
II. Means for dilution of the biomass (1) with a thinner up to a volatile solids content comprised between 0,5% and 1% by volume of the said biomass (1);
III. Means for alkalization of the biomass (1) of item II with an alkaline solution (3) reaching a pH value between 11,0 and 13,0, wherein said biomass (1) of item II and said basic solution (3) are in a weight ratio comprised between 1:20 and 1:80.
IV. Means for hydrolysing the alkalised biomass of item III, for a time comprised between 30 and 90 minutes, at a temperature between 55°C and 80°C and obtaining a hydrolysed biomass (4);
V. Means for neutralisation of the hydrolysed biomass (4) of item IV, by means of CO₂ diffusion from a biogas produced by anaerobic digestion and obtaining a hydrolysed and neutralised biomass (5);
VI. Means for digesting anaerobically, by means of a first anaerobic digester (6), said neutralised and hydrolysed biomass (5) and obtaining a biogas (60), a first digestate (61) and an anaerobic sludge (62).

According to a preferred embodiment, said means for hydrolysing the alkalised biomass allow to carry out an hydrolysis of the said alkalized biomass for a time between 30 and 120 minutes and a temperature between 55°C and 80°C.

Obviously, an expert in the field, in order to meet contingent and specific needs, may make several modifications to the aforementioned embodiments, all of which are falling within the scope of protection of the following claims.

### Examples

The following examples are herewith appended purely for an illustrative purpose and therefore they are not meant to represent a limitation for the present invention.

In order to test the process object of the present invention a pilot plant, able to treat a volume of biomass equal to 0,35 m³, has been realised.

As showed in figure 2, the pilot plant consists of the following essential elements:
iii. An hydrolysis reactor 11 with a volumetric capacity of 0.4 m³ formed by an insulated stainless steel cylindrical tank, an agitator, an electric resistance heating system and sensors for pH and temperature monitoring;
iv. A neutralization column 51 formed by a cylindrical insulated stainless steel tank with a volumetric capacity of 0.4 m³ filled with polyethylene pellet with a porosity of 0.5, such that the operative volume results equal to 0.2 m³, fed from the top with the biomass to be neutralised and from the bottom with the biogas produced by the following anaerobic digestion step;
v. An accumulator tank 52 (accumulator), fed with the biomass to be neutralised that falls in freefall, constituted by an insulated stainless steel cylindrical tank with a volume of 0.1 m³, having a recirculation system that re-injects the material to be neutralized at the top of the neutralization column and sensors for pH and temperature monitoring;
vi. A first anaerobic digester 6, with filtering means, constituted by an insulated stainless steel cylindrical tank, with a volume of 0.75 m³, filled with pumice with a porosity of 0,.5 such that the operative volume is equal to 0.35 m³, having a thermostatically water heating system ensuring a constant temperature of 35 °C.

The inlet biomass object of the present experimental tests, described in the following example, is formed by biologic sludges resulting from a wastewater treatment plant whose characteristics are reported in the table 1.

### Comparative example 1 (without dilution)

A volume equal to 0.35 m³ of biologic sludge characterized in Table 1 has been introduced in the hydrolysis reactor.

**Table 1. Characterization of the biologic sludges used.**

| **Total Solids** | 1.59% | (w/w) |
|---|---|---|
| **Water** | 98.41% | (w/w) |
| **pH** | 6.82 | |
| **Volatile Solids** | 75.95% | (respect to the TS) |
| **Ashes** | 24.05% | (respect to the TS) |
| **Soluble COD** | 82 mg/l | (filtered and centrifugated) |
| **Total COD** | 18690 mg/l | |

The biologic sludge has been alkalised under gentle agitation by addition of a solution of sodium hydroxide 20% w/v, up to a pH value of 12.6.

Subsequently the material has been heated reaching after 1 hour up to a temperature of 68 °C maintaining such conditions for six hours under slight agitation with an energy consumption of 1.31 KWh.

The hydrolysed material displays the characteristics summarised in table 2.

**Table 2. Characterization of the hydrolysed sludge.**

| | | |
|---|---|---|
| **Soluble COD** | 5720 mg/l | (filtered and centrifugated) |
| **Total COD** | 17380 mg/l | |

A volume equal to 0.0875 m3 of hydrolysed sludge has been introduced in the accumulator which feeds the neutralization column from the top, with a descending recirculation flow that returns in the accumulator in freefall.

A neutralisation column contains, under a pressure of about 50 mbar, a biogas volume of 0.21 m3, produced by the anaerobic digestion step, having a percentage of carbon dioxide of 20.46% by volume of said biogas.

After 2 hours of treatment the outlet material presents a value of pH equal to 7.6 and the biogas analysis shows a percentage of carbon dioxide equal to 9.43%.

The accumulator has been fed again with the same volume of hydrolysed sludge and the neutralisation step has been performed again.

After 2 hours the outlet material displays a pH equal to 7.8 and the biogas analysis shows a percentage of carbon dioxide equal to 0.08%.

Once the carbon dioxide has been absorbed, the biogas in the neutralisation column has been substituted with a new volume of biogas to be purified, and the neutralisation step of the hydrolysed sludge has been repeated up to the complete neutralization of 0.35 m3 of the hydrolysed material.

Finally, the neutralised and hydrolysed material has been introduced in the anaerobic digester.

The trigger of the anaerobic digester has been realised through an inoculum, constituted by the liquid fraction of a digestate resulting from an anaerobic digester fed with agricultural and zootechnical wastes, diluted with the hydrolysed sludge with a ratio equal to 1:1.

After 3 months from the start-up the optimal continuous management of the anaerobic digester has been established.

The best performance of the anaerobic digester fed with the neutralised and hydrolysed sludges is displayed in table 3.

**Table 3. Data relating to the best performance of the digester fed with the treated biologic sludge.**

| **Hydraulic retention time (HRT)** | 2 days |
|---|---|
| **Digestate** | |
| COD (digestate) | 3880 mg/l |
| Total Solids % (w/w) digestate | 0.9 |
| Volatile solids % digestate | 31.06 |
| **Biogas produced** | 1.09 m³/m³ |
| **Percentage of CH₄ in the biogas** | 65.53 % (vol/vol) |

The average daily energy consumption for the anaerobic digester operation was of 1.72 KWh.

### Example 2 (with dilution)

A biologic sludge with the same characteristics summarised in the table 1, before being introduced in the hydrolysis reactor, has been diluted with a volumetric ratio of 1:1.2 using a digestate coming from an anaerobic digester, having the characteristics showed in the Table 3, producing a sludge whose characteristics are shown in Table 4.

**Table 4. Characterisation of the biologic sludges diluted with the digestate.**

| | | |
|---|---|---|
| **Total Solids** | 1.21% | (w/w) |
| **Volatile Solids** | 57.72% | (respect to TS) |
| **Soluble COD** | 139 mg/l | (measured on filtered and centrifuged sludge) |
| **Total COD** | 9701 mg/l | |

A volume of 0.35 m3 of said diluted sludge has been introduced in the hydrolysis reactor.

Under slight agitation, the biologic sludge has been alkalised through the addition of a solution of sodium hydroxide 20%, reaching a pH equal to 12.6.

Subsequently the material has been heated reaching after 1 hour at a temperature of 68 °C maintaining such condition for two hours under slight agitation with an energy consumption of 0.72 KWh.

The obtained hydrolysed material displays the properties reported in the table 5. Subsequently, the hydrolysed sludge followed the same neutralisation process described in the comparative example 1 and finally it has been introduced in the anaerobic digester.

**Table 5. Characterisation of the filtered sludge.**

| | | |
|---|---|---|
| **Soluble COD** | 8470 mg/l | (on the filtered and centrifugated sludge) |
| **Total COD** | 9170 mg/l | |

The best yield of the neutralised and hydrolysed sludge is showed in table 6.

**Table 6. Data concerning the best performance of the anaerobic digester fed with biologic sludges diluted using the digestate.**

| **Hydraulic residence time (HRT)** | 2 days |
|---|---|
| **Digestate** | |
| COD | 1480 mg/l |
| Total Solids % (w/w) | 0.7 |
| Volatile Solids % | 20.58 |
| **Biogas produced** | 1.25 m³/ m³ |
| **Percentage of CH₄ in the biogas** | 75.24 % (vol/vol) |

Table 7 displays the comparison of the energy efficiency of the process for the treatment of 0.35 m3 of biologic sludge, with and without the dilution step of the biologic sludge with the digestate coming from the anaerobic digester.

**Table 7. Comparison between the energy efficiency of the process tested on the pilot plant with and without the dilution step of the biologic sludge using the digestate coming from the anaerobic digester and considering a calorific power of methane equal to 9.59 KWh/m3 and a global efficiency of the heating system equal to 0.97.**

| **Heat energy consumption (KWh)** | **Without dilution** | **With dilution** |
|---|---|---|
| Hydrolysis | 1.31 | 1.60 |
| Anaerobic digestion | 3.44 | 7.64 |
| Total | **4.75** | **9.24** |
| **Heat energy produced (KWh)** | **6.65** | **19.45** |
| **Efficiency** | **39.94%** | **105.65%** |

### Example 3 (with dilution)

A biologic sludge with the same characteristics reported in Table 1, before being introduced in the hydrolysis reactor, has been diluted with spring water. The diluted biomass under slightly agitation has been alkalinised by the addition of a solution of sodium hydroxide 20%, reaching a pH value equal to 12.6.

Subsequently the diluted sludge has been heated after 1 hour up to a temperature of 68 °C, maintaining such conditions for two hours under gentle agitation.

Table 8 shows the enhancement of solubilisation efficiency of the solids contained in the inlet biomass measured in terms of ratio between the COD, determined on the clear fraction (supernatant) obtained by filtration, and centrifugation of the hydrolysed material, and the total COD of the hydrolysed material when the dilution percentage changes.

**Table 8. Enhancement of efficiency in the solubilisation of the solids contained in the inlet biomass, when the percentage of dilution changes.**

| **Dilution (%)** | **Volatile Solids % (w/w)** | **Total COD (mg/l)** | **Soluble COD (mg/l)** | **Efficiency (%)** |
|---|---|---|---|---|
| 0 | 1.33 | 19840 | 6510 | 32.81% |
| 20 | 1.05 | 14980 | 7020 | 46.86% |
| 30 | 0.98 | 13930 | 6540 | 46.95% |
| 50 | 0.69 | 8730 | 8008 | 91.73% |

### Example 4

The inlet biomass object of the tests described in the present example is constituted by organic wastes coming from a compost plant, preferably comprising wastes derived from agricultural, horticulture and food preparation, sludges coming from civil and industrial wastewater treatment plant and solid wastes, preventively crushed and mixed, having the characteristics of Table 9.

**Table 9. Characterisation of a waste sample derived from a compost plant.**

| **pH** | 8.00 % |
|---|---|
| **Moisture content** | 73.40% |
| **Residual at 105° C** | 26.60 g/100g |
| **Residual at 600° C** | 6.8 g/100g |

The wastes have been diluted using a digestate coming from an anaerobic digester with the characteristics reported in Table 10.

**Table 10. Characterization of the digestate used as diluent.**

| | |
|---|---|
| **Total Solids** | 0.75% (w/w) |
| **Volatile Solids** | 32.03% (rispetto agli ST) |
| **Total COD** | 1480 mg/l |

In particular, three different dilution ratios have been used: 1:50, 1:25, 1:10.

Table 11 summarises the characterisation of the diluted waste for the different dilution ratios.

**Table 11. Characterization of the diluted wastes with different dilution ratios.**

| **Dilution ratio** | **Total Solids (%)** | **Volatile Solids (%) (compared to TS)** | **Total COD (mg/l)** |
|---|---|---|---|
| 1:50 | 1.26 | 49.70 | 10022 |
| 1:25 | 1.74 | 57.00 | 14616 |
| 1:10 | 3.10 | 65.24 | 26282 |

For each dilution ratio, a volume of 0.35 m3 of the said diluted wastes has been introduced in the hydrolysis reactor.

Under slight agitation, the diluted waste has been alkalised by addition of a solution of sodium hydroxide 20%, reaching a pH equal to 12.6.

Subsequently the material has been heated reaching after 1 hour a temperature of 68 °C maintaining such condition for an hour and half, preferably two hours, under slight agitation with an energy consumption of 0.72 KWh. The obtained hydrolysed material has the characteristics summarised in table 12.

**Table 12. Characterization of the hydrolysed material varying the dilution ratio.**

| **Diluition ratio** | **Total COD (mg/l)** | **Soluble COD (mg/l) (filtered and centrifugated)** |
|---|---|---|
| 1:50 | 9960 | 6970 |
| 1:25 | 14660 | 6438 |
| 1:10 | 26356 | 5797 |

Subsequently, the hydrolysed material has followed the same neutralisation process described in the comparative example 1 and finally it has been introduced in the anaerobic digester.

The best yield obtained with the neutralised and hydrolysed sludge is showed in table 6.

**Table 13. Data concerning the best performance of the digester fed with the treated wastes varying the dilution ratio.**

| | | | |
|---|---|---|---|
| **Residence time (HRT) (day)** | 2 days | | |
| **Diluition ratio** | 1:50 | 1:25 | 1:10 |
| **Digestate** | | | |
| COD | 2534 | 3794 | 6753 |
| Total Solids % (w/w) | 0.51 | 0.82 | 1.66 |
| Volatile Solids % respect to the T S | 0.12 | 0.19 | 0.39 |
| **Biogas produced (m³)** | 0,895 | 0,919 | 0,841 |
| **CH₄ concentration in the biogas (% vol/vol)** | 75.24 | 68.02 | 66.31 |

Table 14 displays the comparison between the process yield evaluated on a residence time (HRT) of 2 days of the digester varying the dilution ratio.

**Table 14. Comparison of the efficiency in terms of yield process tested ion the pilot plant varying the dilution ratio.**

| | | | |
|---|---|---|---|
| **Dilution ratio** | 1:50 | 1:25 | 1:10 |
| **Quantity of treated wastes (Kg)** | 6.86 | 13.46 | 31.82 |
| **m³ CH₄ / waste tonn (HRT = 2 gg)** | 98 | 46 | 18 |

### Example 5

The anaerobic treatment of a volume of 2.8 m³ of the neutralised and hydrolysed material returned a volume of anaerobic sludge of 0.025 m³ taken from the bottom of the anaerobic digester. The anaerobic digester had a brown color and its characteristics are reported in table 15.

**Table 15. Characterisation of the anaerobic sludge at the outlet of the digester.**

| | | |
|---|---|---|
| **COD** | 27220 | mg/l |
| **Total Solids** | 3.49% | (w/w) |
| **Volatile Solids** | 49.55% | (rispetto agli ST) |

From the anaerobic sludge by means of simple freefall percolation process of few hours (6 - 8 hours) made using a sieve with a mesh of 0.25 mm a shovelable solid fraction and a liquid fraction has been obtained having the characteristics reported in the following table.

**Table 16. Characterisation of the solid fraction derived by the percolation process of the anaerobic sludge.**

| | | |
|---|---|---|
| **Total Solids** | 65.66% | (w/w) |
| **Water** | 34.34 % | (w/w) |
| **Volatile Solids** | 48.24% | (on TS) |

**Table 17. Characterization of the liquid fraction obtained from the percolation process of the anaerobic sludge.**

| | | |
|---|---|---|
| **COD** | 7870 | mg/l |
| **Total Solids** | 1.52% | (w/w) |
| **Volatile solids** | 36.08% | (respect to the Total Solids) |

An analysis of the eluate of the solid fraction of the anaerobic sludges for their acceptability in non-hazardous landfills has been carried out. In the following table the results of such analysis are reported.

**Table 18. Analysis of the eluate of the solid phase of the anaerobic sludges for their acceptability in non-hazardous landfill.**

| **Component** | **Value** | **Non-hazardous waste limit DM September 27, 2010** |
|---|---|---|
| Arsenic | <0.05 mg/l | 0.2 |
| Barium | 0.03 mg/l | 10 |
| Cadmium | <0.01 mg/l | 0.1 |
| Total Chrome | <0.01 mg/l | 1 |
| Copper | 2.20 mg/l | 5 |
| Mercury | <0.001 mg/l | 0.02 |
| Molybdenum | 0.10 mg/l | 1 |
| Nikel | 0.11 mg/l | 1 |
| Lead | 0.10 mg/l | 1 |
| Antimony | <0.01 mg/l | 0.07 |
| Selenium | <0.01 mg/l | 0.05 |
| Zinc | 0.29 mg/l | 5 |
| Chlorides | 459.4 mg/l | 2500 |
| Fluorides | 0.4 mg/l | 15 |
| TDS | 265 mg/l | 10000 |
| Sulphates | 26.1 mg/l | 5000 |
| DOC | 210 mg/l | 100 |

The concentration limit of DOC parameter does not apply to this kind of waste because the conditions reported in table 5 of the DM of September 27, 2010 are met. The following table shows the mass balance related to the anaerobic digestion step for the treatment of 2.8 m³ of neutralised and hydrolysed material.

**Table 19. Mass balance of the anaerobic digestion step for the treatment of a 2.8 m³ volume of the biologic sludge.**

| | |
|---|---|
| **Volume of neutralised and hydrolysed material treated (m³)** | 2.8 |
| **Inlet Volatile Solids (Kg)** | 28.14 |
| **Outlet Volatile Solids (digestate) (Kg)** | 5.23 |
| **Outlet Volatile Solids (anaerobic sludge) (Kg)** | 0.43 |
| **Volatile Solids destroyed (Kg)** | 22.48 |
| **Percentange of biodegradability (%)** | 79.89 |
| **Biogas produced (m³)** | 10.37 |
| **Biogas yield Nm³ biogas/t SV** | 461.30 |
| **Residence time (giorni)** | 2 |
| **% of CH₄ before the neutralization step (%)** | 74.33 |

## Claims

1. Process for treating and generating energy from biomass, comprising the step of:
**i.** Providing a biomass (1);
**and characterised by comprising the steps of:**
**ii.** Diluting the biomass (1) with a thinner up to a volatile solids content comprised between 0.5% and 1% of the volume of said biomass (1), wherein the thinner is an aqueous liquid;
**iii.** Alkalizing the biomass (1) of item ii with a basic solution (3), up to a pH comprised between 11.0 and 13.0, wherein said biomass (1) of item ii and said basic solution (3) are in a weight ratio comprised between 1:20 and 1:80;
**iv.** Hydrolysing the alkalised biomass of item iii, for a time comprised between 30 and 90 minutes, at a temperature comprised between 55°C and 80°C, thereby obtaining a hydrolysed biomass (4);
**v.** Neutralising the hydrolysed biomass (4) of item iv, through diffusion of CO₂ from a biogas produced by anaerobic digestion, thereby obtaining a hydrolysed and neutralised sludge (5);
**vi.** Performing anaerobic digestion, through a first anaerobic digestor (6), of said hydrolysed and neutralised sludge (5), thereby obtaining a biogas (60), a first digestate (61), an anaerobic sludge (62),
**wherein**
the biomass (1) is chosen among: biologic sludges, organic wastes, process water, treated or not treated residual sludges from the plants for treating urban, household and industrial wastewater, treated or not treated residual sludges from production processes, and in which the organic wastes are organic wastes resulting from compost plants, comprising wastes derived from agriculture, horticulture and food preparation, sludges resulting from civil and industrial wastewater treatment plants.

2. Process according to claim 1, wherein the thinner is said first digestate (61).

3. Process according to claim 1 , wherein the anaerobic digestion step (vi) is followed by a step of percolating the anaerobic sludge (62), thereby obtaining a liquid phase (63) percolated from said anaerobic sludge (62).

4. Process according to claim 3, wherein the thinner is the liquid phase (63) percolated from the anaerobic sludge (62).

5. Process according to any one of claims 1 to 4, , wherein the first anaerobic digestor (6) is triggered to the anaerobic digestion step (vi) through inoculum of a liquid phase (611) of a second digestate (610), which phase is obtained by means of the following steps:
a) Feeding a second anaerobic digestor (100) with agricultural and zootechnical wastes;
b) Performing the anaerobic digestion of item a, thereby obtaining a second digestate (610);
c) Percolating said second digestate (610), thereby obtaining a liquid phase (611) of the second digestate (610);
d) Performing the inoculum of said liquid phase (611) of the second digestate (610) into said first anaerobic digestor (6), wherein said liquid phase (611) of the second digestate (610) is in a volumetric ratio comprised between 2:1 and 1:2 to the hydrolysed and neutralised sludge (5).

6. Process according to any one of claims 1 to 5, wherein the diffusion of CO₂ while neutralising (v) is made by means of a column reactor (51) in which the biogas (60) is maintained under pressure and is traversed by a descending recirculation current of the hydrolysed biomass (4) until neutralising said hydrolysed biomass (4).

7. Process according to claim 6, wherein said neutralising step (v) occurs by diffusion of CO₂ from the biogas (60) produced from said anaerobic digestion step (vi).

8. Process according to claim 1, wherein the treated or not treated residual sludges from production processes are sludges resulting from oil industry or derived from organic and inorganic chemical processes.

9. Process according to any one of claims from 1 to 2. 6, wherein the biomass (1) is anaerobic sludge (62).

## Patentansprüche

1. Verfahren zur Behandlung und Energiegewinnung aus Biomasse, das die folgenden Stufen umfasst:
**i.** Bereitstellen einer Biomasse (1);
**und dadurch gekennzeichnet, dass es die folgenden Stufen umfasst:**
**ii.** Verdünnen der Biomasse (1) mit einem Verdünner bis zu einem Gehalt an flüchtigen Feststoffen, der zwischen 0,5% und 1% des Volumens der Biomasse (1) liegt, wobei der Verdünner eine wässrige Flüssigkeit ist;
**iii.** Alkalisieren der Biomasse (1) von Punkt ii mit einer basischen Lösung (3) bis zu einem pH-Wert zwischen 11,0 und 13,0, wobei die Biomasse (1) von Punkt ii und die basische Lösung (3) in einem Gewichtsverhältnis zwischen 1:20 und 1:80 vorliegen;
**iv.** Hydrolysieren der alkalisierten Biomasse aus Punkt iii für eine Zeit zwischen 30 und 90 Minuten bei einer Temperatur zwischen 55°C und 80°C, wodurch eine hydrolysierte Biomasse (4) erhalten wird;
**v.** Neutralisieren der hydrolysierten Biomasse (4) aus Punkt iv durch Diffusion von CO2 aus einem durch anaerobe Digestion erzeugten Biogas, wodurch ein hydrolysierter und neutralisierter Schlamm (5) erhalten wird;
**vi.** Durchführen einer anaeroben Digestion des hydrolysierten und neutralisierten Schlamms (5) durch einen ersten anaeroben Digestor (6), wodurch ein Biogas (60), ein erstes Digestat (61) und ein anaerober Schlamm (62) erhalten werden,
**wobei**
die Biomasse (1) ausgewählt wird aus: biologischen Schlämmen, organischen Abfällen, Prozesswasser, behandelten oder nicht behandelten Rückstandsschlämmen aus Anlagen zur Aufbereitung von kommunalem, häuslichem und industriellem Abwasser, behandelten oder nicht behandelten Rückstandsschlämmen aus Produktionsprozessen, und wobei die organischen Abfälle organische Abfälle aus Kompostieranlagen sind, die Abfälle aus der Landwirtschaft, aus dem Gartenbau und der Nahrungsmittelzubereitung umfassen, Schlämme aus zivilen und industriellen Abwasseraufbereitungsanlagen.

2. Verfahren nach Anspruch 1, wobei das Verdünnungsmittel das erste Digestat (61) ist.

3. Verfahren nach Anspruch 1, wobei auf die anaerobe Digestionsstufe (vi) eine Stufe der Perkolation des anaeroben Schlamms (62) folgt, wodurch eine flüssige Phase (63) erhalten wird, die aus dem anaeroben Schlamm (62) perkoliert wird.

4. Verfahren nach Anspruch 3, wobei das Verdünnungsmittel die flüssige Phase (63) ist, die aus dem anaeroben Schlamm (62) perkoliert ist.

5. Verfahren nach einem der Ansprüche 1 bis 4, wobei der erste anaerobe Digestor (6) durch Inokulum einer flüssigen Phase (611) eines zweiten Digestats (610) zur anaeroben Digestionsstufe (vi) ausgelöst wird, wobei die Phase durch die folgenden Stufen erhalten wird:
**a)** Zuführen eines zweiten anaeroben Digestors (100) mit landwirtschaftlichen und zootechnischen Abfällen;
**b)** Durchführen der anaeroben Digestion von Punkt a, wodurch ein zweites Digestat (610) erhalten wird;
**c)** Perkolieren des zweiten Digestats (610), wodurch eine flüssige Phase (611) des zweiten Digestats (610) erhalten wird;
**d)** Durchführen des Inokulums der flüssigen Phase (611) des zweiten Digestats (610) in den ersten anaeroben Digestor (6), wobei die flüssige Phase (611) des zweiten Digestats (610) in einem volumetrischen Verhältnis zwischen 2:1 und 1:2 zu dem hydrolysierten und neutralisierten Schlamm (5) vorliegt.

6. Verfahren nach einem der Ansprüche 1 bis 5, wobei die Diffusion von CO2 während der Neutralisierung (v) mittels eines Säulenreaktors (51) erfolgt, in dem das Biogas (60) unter Druck gehalten wird und von einem absteigenden Rezirkulationsstrom der hydrolysierten Biomasse (4) durchströmt wird, bis die hydrolysierte Biomasse (4) neutralisiert ist.

7. Verfahren nach Anspruch 6, wobei die Neutralisierungsstufe (v) durch Diffusion von CO2 aus dem Biogas (60) erfolgt, das in der anaeroben Digestionsstufe (vi) erzeugt wird.

8. Verfahren nach Anspruch 1, wobei es sich bei den behandelten oder nicht behandelten Restschlämmen aus Produktionsprozessen um Schlämme handelt, die aus der Ölindustrie stammen oder aus organischen und anorganischen chemischen Prozessen gewonnen werden.

9. Verfahren nach einem der Ansprüche 1 bis 6, wobei die Biomasse (1) anaerober Schlamm (62) ist.

## Revendications

1. Procédé de traitement et de production d'énergie à partir de la biomasse, comprenant l'étape consistant à :
**i.** Fournir une biomasse (1) ;
**et caractérisé en ce qu'il comprend en outre les étapes consistant à :**
**ii.** Diluer la biomasse (1) avec un diluant jusqu'à une teneur en solides volatils comprise entre 0,5 % et 1 % du volume de ladite biomasse (1), dans lequel le diluant est un liquide aqueux ;
**iii.** Alcaliniser la biomasse (1) de l'article ii avec une solution basique (3), jusqu'à un pH compris entre 11,0 et 13,0, dans lequel ladite biomasse (1) de l'élément ii et ladite solution basique (3) sont dans un rapport pondéral compris entre 1:20 et 1:80 ;
**iv.** Hydrolyser la biomasse alcalinisée de l'élément iii, pendant une durée comprise entre 30 et 90 minutes, à une température comprise entre 55 °C et 80 °C, de manière à obtenir une biomasse hydrolysée (4) ;
**v.** Neutraliser la biomasse hydrolysée (4) de l'élément iv, par diffusion de CO₂ à partir d'un biogaz produit par digestion anaérobie, ce qui permet d'obtenir une boue hydrolysée et neutralisée (5) ;
**vi.** Effectuer une digestion anaérobie, à travers un premier digesteur anaérobie (6), de ladite boue hydrolysée et neutralisée (5), ce qui permet d'obtenir un biogaz (60), un premier digestat (61), une boue anaérobie (62),
**dans lequel**
la biomasse (1) est choisie parmi : les boues biologiques, les déchets organiques, les eaux de traitement, les boues résiduelles traitées ou non traitées provenant des usines de traitement des eaux résiduaires urbaines, domestiques et industrielles, les boues résiduelles traitées ou non traitées provenant des procédés de production, et dans lesquelles les déchets organiques sont des déchets organiques provenant des usines de compostage, comprenant des déchets provenant de l'agriculture, de l'horticulture et de la préparation alimentaire, les boues provenant des usines de traitement des eaux résiduaires civiles et industrielles.

2. Procédé selon la revendication 1, dans lequel le diluant est ledit premier digestat (61).

3. Procédé selon la revendication 1, dans lequel l'étape de digestion anaérobie (vi) est suivie d'une étape de percolation de la boue anaérobie (62), ce qui permet d'obtenir une phase liquide (63) percolée à partir de ladite boue anaérobie (62).

4. Procédé selon la revendication 3, dans lequel le diluant est la phase liquide (63) percolée à partir de la boue anaérobie (62).

5. Procédé selon l'une quelconque des revendications 1 à 4, dans lequel le premier digesteur anaérobie (6) est déclenché à l'étape de digestion anaérobie (vi) à travers l'inoculum d'une phase liquide (611) d'un second digestat (610), phase qui est obtenue au moyen des étapes suivantes consistant à :
**a)** Alimenter un second digesteur anaérobie (100) en déchets agricoles et zootechniques ;
**b)** Effectuer la digestion anaérobie de l'élément a, ce qui permet d'obtenir un second digestat (610) ;
**c)** Percoler ledit second digestat (610), ce qui permet d'obtenir une phase liquide (611) du second digestat (610) ;
**d)** Effectuer l'inoculum de ladite phase liquide (611) du second digestat (610) dans ledit premier digesteur anaérobie (6), dans lequel ladite phase liquide (611) du second digestat (610) est dans un rapport volumétrique compris entre 2:1 et 1:2 par rapport à la boue hydrolysée et neutralisée (5).

6. Procédé selon l'une quelconque des revendications 1 à 5, dans lequel la diffusion de CO₂ tout en neutralisant (v) est effectuée au moyen d'un réacteur à colonne (51) dans lequel le biogaz (60) est maintenu sous pression et est traversé par un courant de recirculation descendant de la biomasse hydrolysée (4) jusqu'à neutraliser ladite biomasse hydrolysée (4).

7. Procédé selon la revendication 6, dans lequel ladite étape de neutralisation (v) a lieu par diffusion de CO₂ à partir du biogaz (60) produit à partir de ladite étape de digestion anaérobie (vi).

8. Procédé selon la revendication 1, dans lequel les boues résiduelles traitées ou non traitées provenant de procédés de production sont des boues résultant de l'industrie pétrolière ou dérivées de processus chimiques organiques et inorganiques.

9. Procédé selon l'une quelconque des revendications 1 à 6, dans lequel la biomasse (1) est une boue anaérobie (62).
